# EUROPEAN PATENT APPLICATION

(11) **EP 4 790 062 A1**
(43) Date of publication of application: **12.08.2026**
(21) Application number: 24870887.7
(22) Date of filing: 26.09.2024
(51) Int. Cl.: C07K 2/00, C12N 7/02, C12N 7/00, C12Q 1/70, C40B 40/08, C40B 50/06

(54) **PHAGE PARTICLE COMPLEX, PREPARATION METHOD THEREFOR, AND USE THEREOF**

(30) Priority: 28.09.2023 CN 202311282073
(71) Applicant: Pingshan Translational Medicine Center, Shenzhen Bay Laboratory, Shenzhen, Guangdong 518118 (CN); SHENZHEN BAY LABORATORY, Shenzhen, Guangdong 518132 (CN); Peking University Shenzhen Graduate School, Shenzhen, Guangdong 518055 (CN)
(72) Inventor: LI, Zigang, Shenzhen, Guangdong 518118 (CN); YIN, Feng, Shenzhen, Guangdong 518118 (CN); LIU, Na, Shenzhen, Guangdong 518118 (CN)
(74) Representative: Müller-Boré & Partner Patentanwälte PartG mbB
(86) International application number: PCT/CN2024/121477
(87) International publication number: WO 2025/067347

(57) **Abstract**

Disclosed are phage particle complex, preparation process therefor and use thereof, in which the phage particle complex comprising: a peptide displayed on a surface of the phage, a nucleic acid sequence encoding the displayed peptide inside the phage, and a linking compound, wherein the linking compound forms a bicyclic peptide comprising at least three discrete ionic bonds with the peptide.

## Description

### CROSS-REFERENCE TO RELATED APPLICATIONS

The present disclosure claims all the benefits of the Chinese patent application No. 202311282073.X, filed on September 28, 2023 with the China National Intellectual Property Administration, entitled "Phage Particle Composite, Preparation Process Therefor and Use Thereof" and incorporates the entire contents thereof by reference.

### FIELD

The present disclosure generally relates to the field of bioengineering, and more particularly, to phage particle complexes, preparation process therefor and use thereof.

### BACKGROUND

The human body develops a complex and accurate protein-protein interaction (PPIs) network, plays an important role in the life activities of cells, such as signal transduction, cell cycle, immune system, gene regulation, and the like. PPI directly participates in affecting the development of diseases and finally determines whether the disease can be cured. Therefore, targeting intracellular protein-protein interaction is considered to be a very promising therapeutic strategy. Peptide-based drug molecules are considered to be the most effective molecular form of targeting intracellular protein-protein interaction. The peptide drug molecule has a larger active area to form a more complex conformation, can effectively expand the chemical space of the targeting molecule and has a smaller off-target effect relative to the small molecule drug. Similar to biological macromolecules, the peptide molecules also have a higher binding force and selectivity to the target, but the peptide drugs have lower cost and weaker immunogenicity relative to the biological macromolecule drug. Meanwhile, due to its smaller molecular size, it is also easier to penetrate the tissue.

The traditional peptide drug cannot effectively form a complex secondary structure due to the limited number of amino acid residues, and has a high degree of freedom in the physiological solution and has no regular linear state, which not only reduces its specificity and is easily degraded by protease. The cell membrane penetration ability of the peptide drug is not very good. By modifying the peptide by means of chemical means to stabilize the peptide into a conformation with a secondary structure, the stability of the peptide can be increased, the cell membrane penetration ability of the peptide can also be enhanced, and the binding ability of the peptide to the target can also be improved by reducing the entropy change when the peptide is combined with the target.

Peptides are useful in the treatment of various diseases, such as diabetes, allergy, anti-infection, obesity, diagnosis, tumors, arthritis, cardiovascular diseases, and the like, due to their broad active functions. In 2019, the market for oral semaglutide also greatly promotes the enthusiasm of the research and development of peptide drugs. The research and development of peptide drugs is a complex process, and the development of peptide drugs is mainly divided into natural product exploration, rational design, high-throughput screening means, and the like. Natural product exploration involves some degree of contingency and the natural product ligands of most disease-related proteins are not yet clear. The rational design is usually based on the existing protein-protein interaction crystal structure to obtain a peptide sequence targeting the target protein. A series of identification of affinity, stability, cell penetration ability, and the like are carried out. According to the disadvantages optimization and modification are carried out and a peptide molecule with improved affinity, strong stability, suitable physical and chemical properties of the peptide for drug-forming and good biological function is finally obtained. However, due to existing crystal structures and different physical and chemical properties of the peptide sequence, their applicability in drug development is limited. The peptide high-throughput screening technology mainly includes phage display technology, mRNA display technology, and the like. High-throughput screening techniques do not rely on existing protein-protein interactions and can obtain more diverse peptide sequences by screening the number of library diversity above 1 billion, thus providing more potential in the research and development of peptide drugs. In recent years, the peptide molecules obtained by more high-throughput screening means entered clinical studies.

Phage display technology is a widely used high-throughput screening means, which is currently mainly applied to the screening of antibodies and peptides. Adalimumab is a typical case of the technology. Dr. Gregory Winter and Dr. George Smith won the Nobel Chemical Prize in 2018 because of the phage display technology. The peptide displayed in the traditional peptide phage display technology is a linear peptide. The screened linear peptides have poor stability issues and are not easy to enter a cell. Researchers focus on developing a cyclic peptide phage display technology under biological compatibility conditions, which is used for screening peptide drugs. The cyclic peptide drug complement C3 protein inhibitor Pegcetacoplan obtained by the cyclic peptide phage display technology is approved by the FDA for the treatment of paroxysmal nocturnal hemoglobinuria (PNH). The bicyclic peptide phage display technology developed by Dr. Winter has the potential for the development of peptide drugs. A plurality of peptide molecules entered phase II/III clinical trials. Moreover, Mr. WU Chuanliu of Xiamen University developed bicyclic or tricyclic phage display peptide technology with different structures such as CXC, CPPC, CPXXC. A method for screening a covalent ring peptide drug through phage display technology is developed by Dr. Matthew Bogyo of Stanford University for the first time, which enriches the type of peptide phage display technology. The cyclic peptide has more druggability than the linear peptide, which also means that the cyclic peptide phage display technology further expands the applicability of the linear peptide phage display technology.

### SUMMARY

In one aspect, the present disclosure relates to a phage particle complex, comprising: a peptide displayed on a surface of the phage, a nucleic acid sequence encoding the displayed peptide inside the phage, and a linking compound, wherein the linking compound forms a bicyclic peptide comprising at least three discrete ionic bonds with the peptide.

In another aspect, the present disclosure relates to a peptide library, comprising the phage particle complex of the present disclosure.

In still another aspect, the present disclosure relates to a process for preparing a phage particle complex, comprising forming a bicyclic peptide containing at least three discrete ionic bonds by a peptide displayed on a surface of the phage with a linking compound.

In still another aspect, the present disclosure relates to phage particle complex prepared by the process comprising forming a bicyclic peptide containing at least three discrete ionic bonds by a peptide displayed on a surface of the phage with a linking compound.

In still yet another aspect, the present disclosure relates to use of the peptide library of the present disclosure in manufacture of a peptide inhibitor.

In still yet another aspect, the present disclosure relates to a nucleotide sequence selected from the group consisting of SEQ ID NO:1 to SEQ ID NO:8.

### BRIEF DESCRIPTION OF THE DRAWINGS

Fig. 1 shows the formation of a bicyclic peptide by alkylation of methionine of a linear peptide under weak acid conditions, wherein A shows the schematic diagram of the formation of a bicyclic peptide by alkylation of methionine of a linear peptide under weak acid conditions; and B shows the efficiency of the formation of a bicyclic peptide by the alkylation of methionine of a linear peptide under weak acid conditions over different times.
Fig. 2 shows the purification diagram of the phage coat protein pIII of the present disclosure.
Fig. 3 shows the first-order mass spectrum of the reaction of the phage coat protein pIII of the present disclosure and TBMB, wherein Fig. A shows the first-order mass spectrum results of the coat protein of the linear peptide displayed at the N-terminus; and Fig. B shows the first-order mass spectrum results of the coat protein after the N-terminal peptide is cyclized.
Fig. 4 shows the schematic diagram of a semi-random peptide structure of phage display of the present disclosure.
Fig. 5 shows the schematic diagram of construction of recombinant plasmids in the phage display technology of the present disclosure.
Fig. 6 shows the results of the quality identification of *E. coli* library obtained by the phage display technology of the present disclosure, wherein Fig. A shows the proportion of sequences that are sequenced; Fig. B shows the proportion of different M contained in the semi-random library; Fig. C shows the content of A, T, C, and G in each position of 10000 randomly selected sequences; and Fig. D shows comparison between the 19 amino acids (excluding M) translated from 10000 sequences and the theoretical amino acid ratios.
Fig. 7 shows a portion of the high-throughput sequencing analysis of the homologous peptide molecular groups of the present disclosure.
Fig. 8 shows the enzyme inhibitory activity IC₅₀ of the screened peptides W5-3M and C5-3M of the present disclosure.
Fig. 9 shows the affinity of a screened peptide targeting STAT3 of the present disclosure.
Fig. 10 shows the affinity of a screened peptide targeting ERα of the present disclosure.

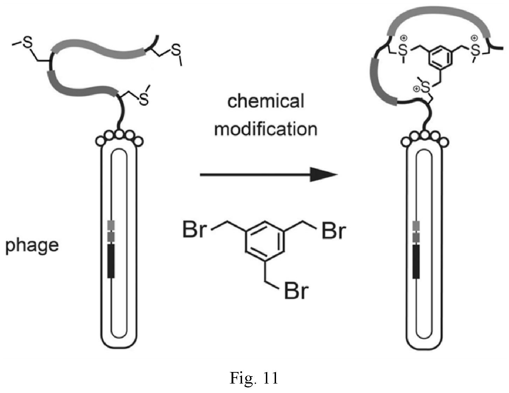

Fig. 11 shows the schematic diagram of the phage display technology of a sulfonium stapled bicyclic peptide of the present disclosure.

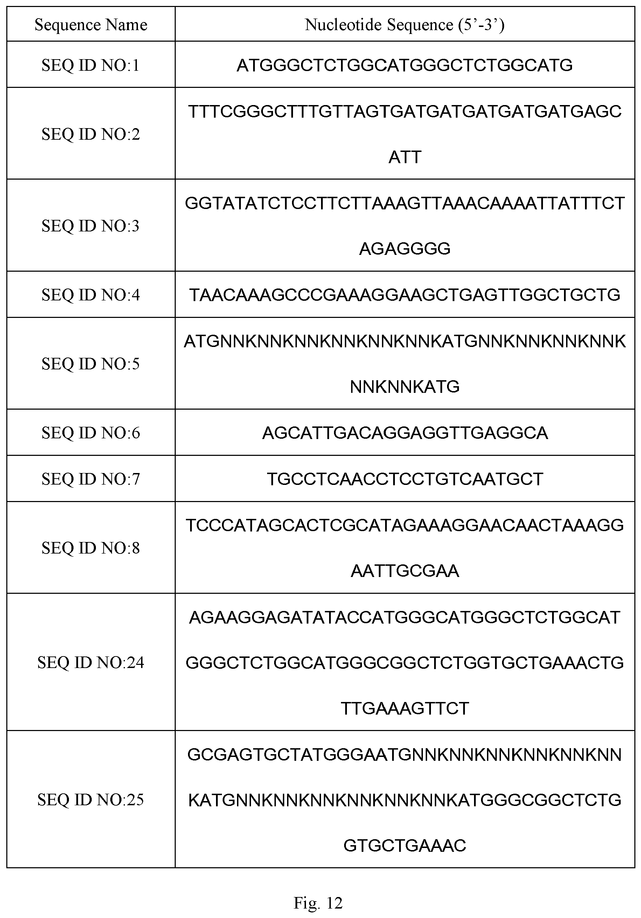

Fig. 12 shows the nucleotide sequences of SEQ ID NO:1 to SEQ ID NO:8, SEQ ID NO:24 and SEQ ID NO:25 of the present disclosure.

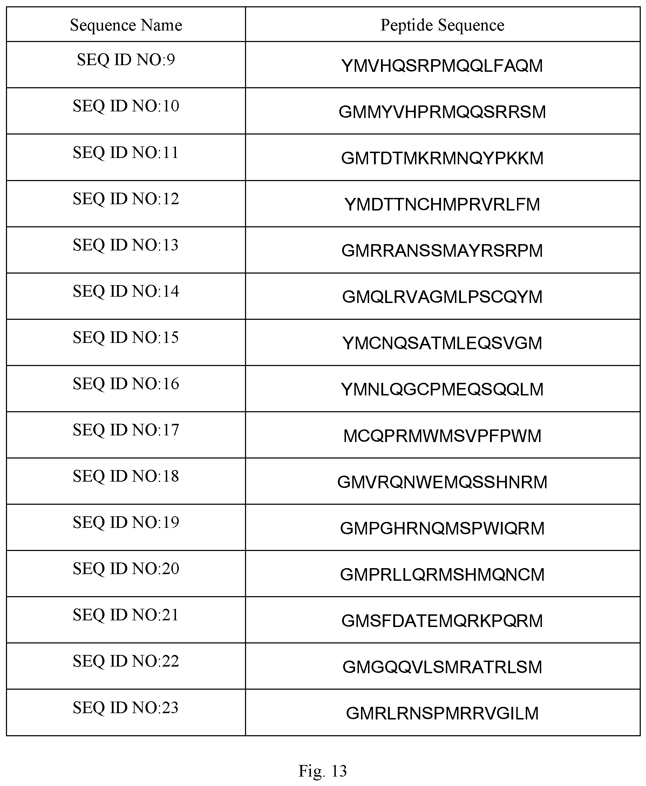

Fig. 13 shows the peptide sequences of SEQ ID NO:9 to SEQ ID NO:23 of the present disclosure.

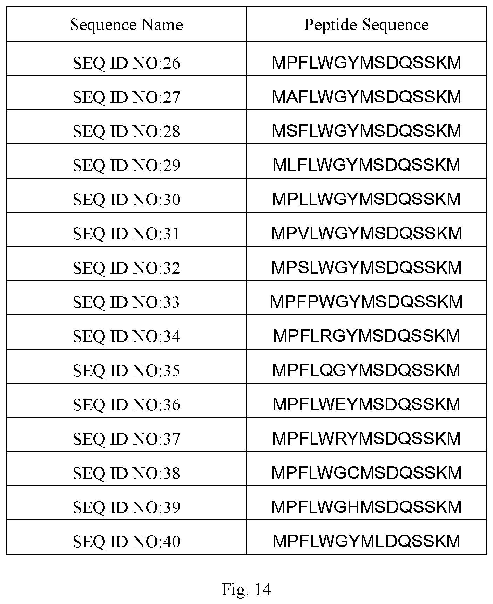

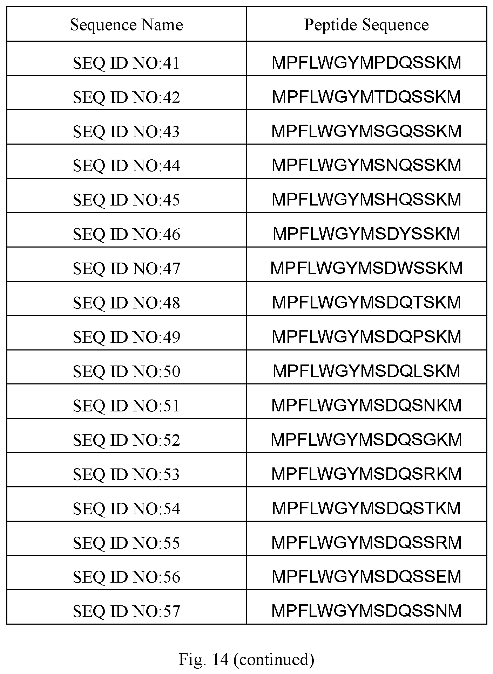

Fig. 14 shows the peptide sequences of SEQ ID NO:26 to SEQ ID NO:57 of the present disclosure.

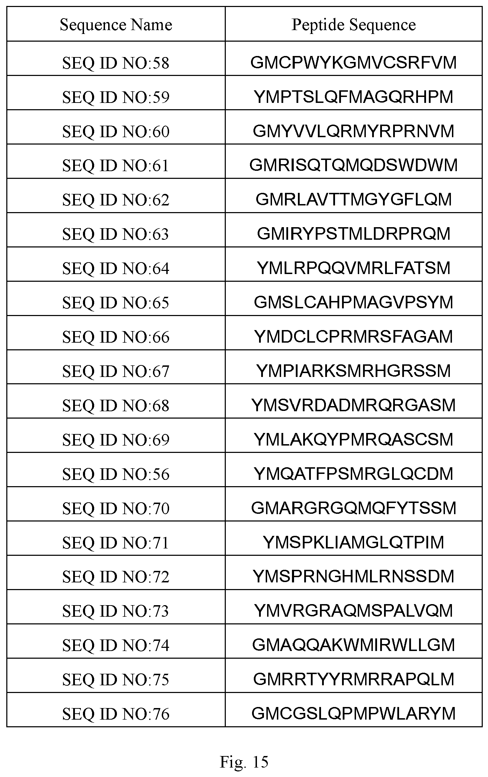

Fig. 15 shows the peptide sequences of SEQ ID NO:58 to SEQ ID NO:76 of the present disclosure.

### DETAILED DESCRIPTION

In the following description, certain specific details are included to provide a thorough understanding of the various disclosed embodiments. However, one skilled in the art will recognize that embodiments may be carried out without one or more of these specific details, but with other methods, components, materials and the like.

Unless otherwise claimed in this application, throughout the specification and the appended claims, the terms "comprise", "comprising," "contain" and "having" are to be construed in an open, inclusive sense, i.e., "including, but not limited to".

As used in this disclosure and the appended claims, the singular reference without an indication of quantity includes the plural reference unless the context clearly dictates otherwise.

Reference throughout this specification to "one embodiment" or "an embodiment" or "in another embodiment", or "in some embodiments" means that a particular referent feature structure or characteristic described in connection with the embodiments is included in at least one embodiment. Therefore, the appearance of the phrases "in one embodiment", "in the embodiment", "in another embodiment", or "in some embodiments" in various places throughout this specification are not necessarily all referring to the same embodiment. Moreover, the particular features structures or characteristics can be combined in any suitable manner in one or more embodiments.

It should be noted that, as used in this specification and the appended claims, the singular forms "a", "an" and "the" comprise plural referents unless the context clearly stated otherwise. Therefore, for example, a mentioned composition comprising "a closed-loop peptide comprising at least two methionines and at least two sulfonium centers" comprises one closed-loop peptide comprising at least two methionines and at least two sulfonium centers, or two or more closed-loop peptides comprising at least two methionines and at least two sulfonium centers.

### DEFINITION

As used herein, the term "phage" refers to the general term of viruses that infected microorganisms such as bacteria, fungi, algae, actinomycetes, spirochetes and the like. Because some of them can cause lysis of the host bacteria, they are called phages.

As used herein, the term "phagemid" refers to a plasmid with a filamentous phage replication initiation point.

As used herein, the term "plasmid" refers to a class of nucleic acid molecules that are inherent in biological cells and can be autonomously replicated independently of the host chromosome and are stably inherited.

As used herein, the term "phage display technology" refers to a technique for fusing a protein or peptide of an exogenous to a capsid protein and displaying the protein or peptide on a phage surface while maintaining the biological activity and spatial conformation of the exogenous protein or peptide.

As used herein, the term "ionic bond" refers to a chemical bond formed after ions are lost or obtained by two or more atoms or chemical groups.

As used herein, the term "residue" refers to a portion remaining after the dehydration condensation of amino and carboxyl groups in a protein or peptide.

As used herein, the term "peptide" refers to a polymer formed by covalent attachment of two or more amino groups through a peptide bond. It is novel nutrient between macromolecular proteins and amino acids with highest activity, most easily absorbed and highly effective in physiological functions. It is generally composed of L-type α-amino acids.

As used herein, the term "peptide bond" refers to an amide bond formed by the condensation of the carboxyl group of one amino acid with the amino group of another amino acid and the removal of one molecule of water.

As used herein, the term "natural amino acid" refers to an amino acid synthesized by biosynthesis.

### SPECIFIC EMBODIMENTS

In one aspect, the present disclosure relates to a phage particle complex, comprising: a peptide displayed on a surface of the phage, a nucleic acid sequence encoding the displayed peptide inside the phage, and a linking compound, wherein the linking compound forms a bicyclic peptide comprising at least three discrete ionic bonds with the peptide.

In some embodiments, the linking compound that can be used in the present disclosure has molecular symmetry corresponding to the number of the ionic bonds.

In some embodiments, the linking compound that can be used in the present disclosure has triple molecular symmetry.

In some embodiments, each ionic bond is formed by a separate amino acid residue on a peptide.

In some embodiments, the linking compound that can be used in the present disclosure is linked to the peptide by three discrete ionic bonds.

In some embodiments, the linking compound that can be used in the present disclosure comprises a chemical group of a rigid structure.

In some embodiments, the linking compound that can be used in the present disclosure comprises a benzene ring structure.

In some embodiments, the linking compound that can be used in the present disclosure comprises a cycloalkene structure.

In some embodiments, exemplary examples of the linking compound that can be used in the present disclosure include, but are not limited to, 1,3,5-tribromobenzene, 1,3,5-tris(bromomethyl)-2,4,6-triethylbenzene and 1,3,5-tris(bromomethyl)cyclohexene.

In some embodiments, the peptide that can be used in the present disclosure comprises at least three methionine residues.

In some embodiments, a discrete ionic bond is formed by linking the compound with methionine in the peptide.

In some embodiments, a peptide sequence that can be used in the present disclosure comprises MXmMXnM, wherein X represents a natural amino acid residue, M represents methionine, m is an integer selected from 2 to 20, and n is an integer selected from 2 to 20.

In some embodiments, R is selected from the group consisting of glycine (G), alanine (A), valine (V), leucine (L), isoleucine (I), methionine (M), proline (P), tryptophan (W), serine (S), tyrosine (Y), cysteine (C), phenylalanine (F), asparagine (N), glutamine (Q), threonine (T), aspartic acid (D), glutamic acid (E), lysine (K), arginine (R) and histidine (H) residues.

In some embodiments, m is an integer selected from 3 to 10.

In some embodiments, m is an integer selected from 3 to 6.

In some embodiments, n is an integer selected from 3 to 10.

In some embodiments, n is an integer selected from 3 to 6.

In some embodiments, exemplary examples of peptides that can be displayed on the surface of the phage of the present disclosure include, but are not limited to, SEQ ID NO:9 to SEQ ID NO:23.

In some embodiments, the bicyclic peptide has a structure of: 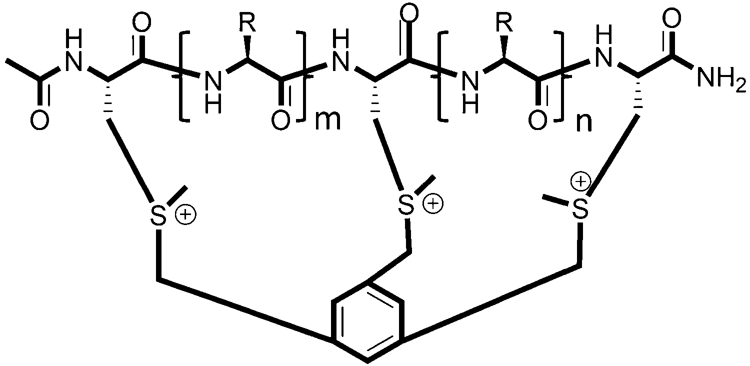 wherein R is selected from a side chain of 20 natural amino acids, m is an integer selected from 2 to 20, and n is an integer selected from 2 to 20.

In some embodiments, R is selected from the group consisting of glycine (G), alanine (A), valine (V), leucine (L), isoleucine (I), methionine (M), proline (P), tryptophan (W), serine (S), tyrosine (Y), cysteine (C), phenylalanine (F), asparagine (N), glutamine (Q), threonine (T), aspartic acid (D), glutamic acid (E), lysine (K), arginine (R) and histidine (H) residues.

In some embodiments, m is an integer selected from 3 to 10.

In some embodiments, m is an integer selected from 3 to 6.

In some embodiments, n is an integer selected from 3 to 10.

In some embodiments, n is an integer selected from 3 to 6.

In another aspect, the present disclosure relates to a peptide library, comprising the phage particle complex of the present disclosure.

In still another aspect, the present disclosure relates to a process for preparing a phage particle complex, comprising forming a bicyclic peptide containing at least three discrete ionic bonds by a peptide displayed on a surface of the phage with a linking compound.

In some embodiments, the bicyclic peptide is formed in a polar solvent.

In some embodiments, exemplary examples of the polar solvent that can be used in the present disclosure include, but are not limited, an aqueous solution of acetonitrile, an aqueous solution of methanol and any mixture thereof.

In some embodiments, the bicyclic peptide is formed at about 4 to 40°C.

In some embodiments, the bicyclic peptide is formed at a pH of about 3 to 10.

In some embodiments, the reaction time of the peptide and the linking compound to form the bicyclic peptide is from 1 to 36 hours.

In still another aspect, the present disclosure relates to phage particle complex prepared by the process comprising forming a bicyclic peptide containing at least three discrete ionic bonds by a peptide displayed on a surface of the phage with a linking compound.

In some embodiments, the phage particle complex prepared by the process of the present disclosure further comprises a nucleic acid sequence encoding the peptide.

In some embodiments, in the phage particle complex prepared by the process of the present disclosure, the peptide comprises at least three methionine residues.

In some embodiments, in the phage particle complex prepared by the process of the present disclosure, a discrete ionic bond is formed by linking the compound with methionine in the peptide.

In still yet another aspect, the present disclosure relates to use of the peptide library of the present disclosure in manufacture of a peptide inhibitor.

In some embodiments, exemplary examples of a peptide inhibitor prepared by the peptide library of the present disclosure include, but are not limited to, an infectious disease peptide inhibitor, a tumor peptide inhibitor, an endocrine disease peptide inhibitor and a digestive system disease peptide inhibitor.

In some embodiments, exemplary examples of a peptide inhibitor prepared by the peptide library of the present disclosure include, but are not limited to, a SARS-CoV-2 PLpro peptide inhibitor, a breast cancer peptide inhibitor, a leukemia peptide inhibitor, a nutritional and metabolic disease peptide inhibitor and an enteritis peptide inhibitor.

In some embodiments, exemplary examples of a peptide inhibitor prepared by the peptide library of the present disclosure include, but are not limited to, SEQ ID NO:9 to SEQ ID NO:23 and SEQ ID NO:26 to SEQ ID NO:76.

In still yet another aspect, the present disclosure relates to a nucleotide sequence selected from the group consisting of SEQ ID NO:1 to SEQ ID NO:8.

The present disclosure will hereinafter be explained in detail in order to better understand the aspects of the present application and its advantages. However, it is to be understood that the following examples are non-limiting and merely illustrative of certain embodiments of the present disclosure.

### EXAMPLES

The reagents and devices used in the embodiments of the present disclosure are both conventional and commercially available. For example:

| Name | CAS | Manufacturer |
|---|---|---|
| 1,3,5-tribromobenzene *E. coli* TG1 | 18226-42-1 | U.S. Sigma-Aldrich Corp (Sigma) Shanghai Beyotime Biotechnolgy Co., Ltd |
| pCANTAB 5E | | YouBio Technology Co., Ltd |
| M13K07 | | YouBio Technology Co., Ltd |
| Streptavidin magnetic beads | | Thermo Fisher Scientific |
| Biotin Sulfo-NHS-LC-Biotin | | Thermo Fisher Scientific |
| Tosyl-activated magnetic beads | | Thermo Fisher Scientific |
| Avidin ligand | | Thermo Fisher Scientific |
| PEG8000 | | Shanghai Sangon Biotech Co., Ltd |

### Example 1

### Preparation, Separation and Purification of Sulfonium Stapled Bicyclic Peptides

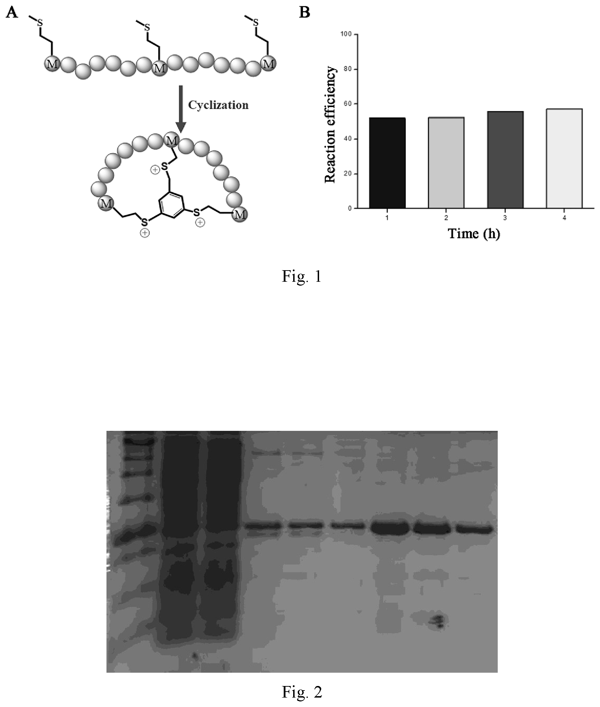

A peptide was prepared according to an amino acid sequence. The core steps for preparing a sulfonium stapled bicyclic peptide are as follows (taking the peptide sequence: Ac-Y-M-G-S-G-G-S-G-S-G-G-S-G-M-NH₂ as an example):
(1) Solid-phase synthesis of peptide: To the peptide formation tube, was weighed and added Rink amide MBHA resin and was added dichloromethane (DCM). Rink amide MBHA resin was swollen with nitrogen for 30 min. Morpholine in N,N-dimethylformamide (DMF) (50% (v/v)) was added. The resin was swollen with nitrogen for 30 min to remove the Fmoc protecting group. After washing the resin with DMF and DCM alternately, the prepared Fmoc-protected amino acid (5eq, 0.4M, DMF) solution, 6-chlorobenzotriazole-1,1,3,3-tetramethyluronium hexafluorophosphate (HCTU) (5eq, 0.38M, DMF) solution, N,N-diisopropylethylamine (DIPEA) (10eq) were mixed evenly. The resultant mixture was added into the resin. The resin was swollen with nitrogen for 1 h. The reaction solution was extracted. The resin was washed according to the above method and then subjected to the next operation. The following amino acids are assembled in the same manner as described above. The N-terminus of the peptide was acetylated with acetic anhydride and DIPEA (1:1.8, dissolved in DCM) for 30 min (twice).
(2) Double alkylation addition reaction: After the linear peptide comprising three methionine was prepared on the solid phase, to the EP tube were added 50 mg of the prepared resin and 1 mL of shearing solution (trifluoroacetic acid/triisopropylsilane/water/EDT (v:v:v=9.5:0.25:0.25)). The resultant mixture was sheared for 1h. The shearing solution was blown to dryness with nitrogen. 0.5mL of cold diethyl ether was added for precipitation for 2 min. The mixture was centrifuged to remove the supernatant. Diethyl ether was vaporized from he precipitated peptide in the air. The peptide was then dissolved in 30% acetonitrile/water solution to form a peptide solution with a concentration of about 10mM. To the solution was added 1% by volume of formic acid (pH is about 3). 1.2 peptide equivalents of alkylating agent were weighed and dissolved in in about one-tenth of the solvent volume of DMF. The mixture was added to the peptide solution. The resultant mixture was reacted for 24 h in a shaker (Fig. 1).
(3) Identification of peptide purification and reaction efficiency: The peptide purified with high performance liquid chromatography was used. The product conversion rate was calculated according to the ratio of the integral of the peak area of the product peak on the high performance liquid chromatography to the integral of the total peak area of the raw material and the product.

### Example 2

### Sulfonium Stapled Bicyclization of Linear Peptide Displayed at N-Terminus of Phage Coat Protein

### 1. Protein sequence design

On the basis of the directed evolution-mutated C-free phage coat protein pIII protein of Professor Francz X Schmid (J Mol Biol. 2005, 2;354(3):666-78), the pIII protein sequence of the new phage coat protein was designed. The domain of D1-D2 of the pIII protein was truncated. M in the protein was mutated to A. The new protein sequence was as follows:

AETVESSLAKSHIEGSFTNVWKDDKTLDWYANYEGILWKATGVVVITGDE
TQVYAIWVPVGLAIPENEGGGSEGGGSEGGGSEGGGTKPPEYGDTPIP
GYIYINPLDGTYPPGTEQNPANPNPSLEESHPLNTFAFQGNRFRNRQGAL
TVYTGTVTQGTDPVKTYYQYTPVSSKAAYDAYWNGKFRDVAFHSGFNE
DPLVAEYQGQLSYLPQPPVNAHHHHHH

### 2. Construction of recombinant plasmid pET28a pIII

According to the protein sequence designed above, codon optimization of prokaryotic cell (E. coli) was carried out. The DNA gene sequence encoding the new protein was determined. The sequence was as follows:

GCTGAAACTGTTGAAAGTTCTTTAGCAAAATCTCATATAGAAGGTTCAT
TTACTAACGTCTGGAAAGACGACAAAACTTTAGATTGGTACGCTAACT
ATGAGGGCATTCTGTGGAAGGCTACAGGCGTTGTGGTTATTACTGGT
GACGAAACTCAGGTTTACGCTATATGGGTTCCTGTTGGGCTTGCTATC
CCTGAAAATGAGGGTGGTGGCTCTGAGGGTGGCGGTTCTGAGGGTG
GCGGTTCTGAGGGTGGCGGTACTAAACCTCCTGAGTACGGTGATACA
CCTATTCCGGGCTATATTTATATCAACCCTCTCGACGGCACTTATCCG
CCTGGTACTGAGCAAAACCCCGCTAATCCTAATCCTTCTCTTGAGGAG
TCTCATCCTCTTAATACTTTCGCGTTTCAGGGTAATAGGTTCCGAAATA
GGCAGGGTGCATTAACTGTTTATACGGGCACTGTTACTCAAGGCACTG
ACCCCGTTAAAACTTATTACCAGTACACTCCTGTATCATCAAAAGCCG
CGTATGACGCTTACTGGAACGGTAAATTCAGAGACGTCGCTTTCCATT
CTGGCTTTAATGAGGATCCATTAGTTGCTGAATATCAAGGCCAATTGT

CTTACCTGCCTCAACCTCCTGTCAATGCTCATCATCATCATCATCAC

The preparation steps of the DNA gene sequence encoding the new protein were: scheme design, primer synthesis, oligonucleotide splicing, gene assembly and quality inspection of finished product.

Primer synthesis: A small fragment primer with an overlapping area of 40 to 200 nt was synthesized by the Oligo chemical synthesis manner.

Scheme design: The sequence was further analyzed. According to the characteristics of the codon degeneracy, by replacing partially deoxynucleotides, the occurrence of a highly repeated sequence was avoided, the complex secondary structure was reduced and the GC content was ensured to be 40 to 60.

Oligonucleotide splicing: A single-stranded primer was spliced into a double-stranded gene fragment with the overlapping PCR technique. For a gene with no complex secondary structure within 3 Kb, full-length assembly of the fourth step could be directly carried out; if the full length of the gene exceeds 3 Kb, fragment assembly needed to be carried out.

Gene assembly: By assembly technologies such as seamless cloning, enzyme digestion, and the like, the fragments were further spliced into a full-length sequence and inserted into a pUC series vector. After ligation conversion, cloning and screening, sequencing verification and mutation repair, a completely correct recombinant vector was obtained. For a gene of more than 3 Kb, the gene needed to be divided into a plurality of fragments and inserted into a plurality of pUC series vectors. After ligation conversion, cloning and screening, sequencing verification and mutation repair, a completely correct recombinant vector was obtained.

Quality inspection of finished product: Final quality detection was carried out on the finished product by sequencing verification, restriction digestion and gel electrophoresis, and the like.

### 3. Primer design: new gene was ligated into pET28a plasmid by homologous recombination

The specific primer design steps comprised: designing a primer with the primer design software CE Design.

The Fdg3p-MG-XIN-F primer comprised three parts: homologous arm (AGAAGAGATATACC, 15bp, homologous to the linearized vector fragment pET28a plasmid) + exogenous DNA (ATGGGCATGGGCTCTGGCATGGGCTCTGGCATGGGCGGCTCTGGT) + specific primer (GCTGAAACTGTTGAAAGTTCT, homologous to pIII gene).

The fdg3p-3M-R primer comprised two parts: homologous arm (TTTCGGGCTTTGTTA, 15bp, homologous to linearized vector fragment pET28a plasmid) + specific primer (GTGATGATGATGATGAGCATT, homologous to pIII gene).

Pet28-3M-F and Pet28-3M-R were a pair of primers for reverse PCR amplification of linearized vector pET28a plasmid.

**Table 1. Primer sequences for construction of recombinant plasmids**

| Primer | Sequence | Sequence Name |
|---|---|---|
| Fdg3p-MG-XIN-F | AGAAGGAGATATACCATGGGCATGGGC TCTGGCATGGGCTCTGGCATGGGCGGC TCTGGTGCTGAAACTGTTGAAAGTTCT | SEQ ID NO:24 |
| fdg3p-3M-R | TTTCGGGCTTTGTTAGTGATGATGATGA TGATGAGCATT | SEQ ID NO:2 |
| Pet28-3M-F | GGTATATCTCCTTCTTAAAGTTAAACAAA ATTATTTCTAGAGGGG | SEQ ID NO:3 |
| Pet28-3M-R | TAACAAAGCCCGAAAGGAAGCTGAGTT GGCTGCTG | SEQ ID NO:4 |

### 4. Amplification of gene fragments and linear plasmid fragments with PCR

**Table 2. Standard PCR amplification system**

| Component | Volume (µL) |
|---|---|
| Primestar MAX | 25 |
| Upstream primer (10µM) | 1 |
| Downstream primer (10µM) | 1 |
| Template DNA | 1 |
| ddH₂O | 22 |
| In total | 50 |

**Table 3. PCR reaction conditions**

| | | |
|---|---|---|
| 98°C | 5min | |
| 98°C | 10s | 30 to 35 cycles |
| 55°C | 5s | |
| 72°C | 10s (gene)/1min (plasmid) | |
| 72°C | 10min | |
| 4°C | Maintain | |

### 5. Agarose gel electrophoresis identification and purification of PCR products

1% agarose gel was prepared. The PCR sample and 10 × loading buffer were mixed homogeneously. The mixture was added into sample wells, subject to current at 120V for 30min, observed and photographed.

It was confirmed that the size of the PCR product was consistent with the prediction result. The purification was carried out. The PCR product was recovered by the gel-cutting recovery kit to obtain the pure PCR product.

### 6. Homologous recombination construction of recombinant plasmids. The system was as follows:

**Table 3. Homologous recombination system**

| Component | Volume (µL) |
|---|---|
| Gene (40ng) | Determined based on concentration |
| pET28a (120ng) | Determined based on concentration |
| Exnase II | 1 |
| 5 × CE buffer | 2 |
| ddH₂O | To 10µL |
| In total | 10 |

### 7. Recombinant plasmid transformation and cloning picking

Plasmid constructed by homologous recombination was transformed into competent cells and cultured overnight at 37°C. Then, the monoclonal was picked up for sequencing to confirm whether the construction was successful.

### 8. Recombinant protein-induced expression

After the recombinant plasmid was confirmed to be successfully constructed, the plasmid was transformed into the expression strain BL21 pLysS. Monoclonal bacteria were picked to 5ml of LB medium (50µg/mL kanamycin). The medium was cultured overnight at 37°C and 200rpm. The culture solution was then transferred to 1000mL of LB medium (50µg/mL kanamycin). The medium was cultured at 37°C and 200rpm until OD₆₀₀ was 0.4 to 0.6. The final concentration of 0.5 mM IPTG solution was added into the medium. The resultant medium was cultured overnight (12-18h) at 16°C and 150rpm. After induction was completed, . the medium was centrifuged at 4°C and 5000rpm for 10min. The bacteria were collected and cryopreserved at -80°C.

### 9. Purification of recombinant protein

The collected bacteria were added to 30 to 50 mL of protein purification buffer A. The resultant mixture was vortexed, blown and fully re-suspended. The ultrasonic crushing instrument was used to crush. After ultrasonic crushing, centrifugation was carried out at 4°C and 14,000 rpm for 30min. The supernatant was taken. The product was filtered with 0.22µm or 0.45µm filter membrane to obtain the protein supernatant. Then affinity chromatography was carried out on 5 mL of His Trap^{TM} FF Crude column. Separation was carried out with Superdex^{™}200 10/300 GL molecular sieves. Electrophoresis analysis of different components were carried out with SDS-PAGE (Fig. 2).

### 10. Sulfonium cyclization of peptides of phage coat protein at N-terminus

The purified phage coat protein pIII was reacted with 1,3,5-tribromomethylbenzene (TBMB) at 37°C for 2 to24h. The reaction system was as follows:

**Table 4. Reaction system of phage coat protein and TBMB**

| Component | Volume (µL) |
|---|---|
| pIII | (Final concentration 50µM) |
| TBMB | (Final concentration 100µM) |
| PBS (pH=6.0) | 140 |
| Acetonitrile | 60 |
| In total | 200 |

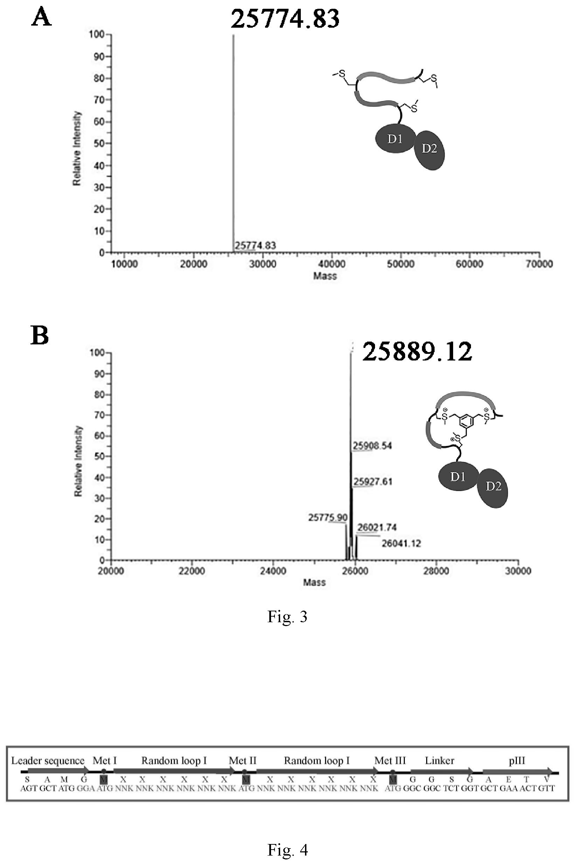

After the reaction, the reaction solution was centrifuged at 10,000rpm for 30min using the ultrafiltration centrifuge tube. ddH₂O was added. The ultrafiltration was repeated three times. The first-order primary mass spectrum was use to determine whether three M of the N-terminus of the protein could be reacted with TBMB (Fig. 3).

### Example 3

### Construction of Sulfonium Stapled Bicyclic Peptide of Phage Particle Complex

Sulfonium stapled bicyclic peptides of phage particle complex were constructed, wherein a random peptide sequence was introduced between the signal peptide of the phage coat protein pIII and the sequence of pIII at N-terminus (Fig. 4). The specific operation was as follows:

### 1. Primer design

The primers shown in Table 5 were designed with pCANTAB 5E as a template.

The new gene was ligated into the pCANTAB 5E plasmid by homologous recombination.

The specific primer design steps comprised: designing a primer with primer design software CE Design.

NNK-N1N2-F primer comprised three parts: homologous arm (GCGAGTGCTATGGGA, homologous to linearized vector fragment pCANTAB plasmid) + introduction of exogenous DNA (ATGNNKNNKNNKNNKNNKNNKNNKNNKNNKNNKNNKNNKATG) + specific primer (GGCGGCTCTGGTGCTGAAAC, homologous to pIII gene).

NNK-N1N2-R primer comprised two parts: homologous arm (AGCATTTGACAGGAGG, 15bp, homologous to linearized vector fragment pCANTAB 5E plasmid) + specific primer (TTGAGGCA, homologous to pIII gene).

P1-F and P1-R were a pair of primers for reverse PCR amplification of linearized vector pCANTAB 5E plasmid.

**Table 5. Primer sequences of recombinant phagemid**

| Primer | Sequence | Sequence Name |
|---|---|---|
| NNK-N1N2-F | GCGAGTGCTATGGGAATGNNKNNKNNK NNKNNKNNKATGNNKNNKNNKNNKNNKN NKATGGGCGGCTCTGGTGCTGAAAC | SEQ ID NO:25 |
| NNK-N1N2-R | AGCATTGACAGGAGGTTGAGGCA | SEQ ID NO:6 |
| P1-F | TGCCTCAACCTCCTGTCAATGCT | SEQ ID NO:7 |
| P1-R | TCCCATAGCACTCGCATAGAAAGGAACA ACTAAAGGAATTGCGAA | SEQ ID NO:8 |

2. The recombinant plasmid in Example 2 was used as a template. The inserted fragment was amplified by PCR reaction and the product was purified. The linear fragment of the pCANTAB 5E plasmid was amplified by PCR reaction and recovered by gel extraction.

3. The recombination reaction was carried out with ConExpress II kit. The optimal amount of the cloning vector was 0.03pmol and the optimal molar ratio of the cloning vector to the inserted fragment was 1:2, that is, the optimal amount of the inserted fragment was 0.06pmol. The reaction system was as follows:

**Table 6. Homologous recombination system**

| Component | Volume (µL) |
|---|---|
| Gene (400ng) | Determined based on concentration |
| pCANTAB 5E linear fragment (1200ng) | Determined based on concentration |
| Exnase II | 10 |
| 5 × CE buffer | 20 |
| ddH₂O | To 100µL |
| In total | 100 |

After the reaction system was prepared, the reaction system was slightly flicked and then centrifuged. The reaction was carried out at 37°C for 30min. The vector template in the recombinant product was removed with DpnI enzyme.

### 4. Preparation of primary E. coli library by electrical conversion method

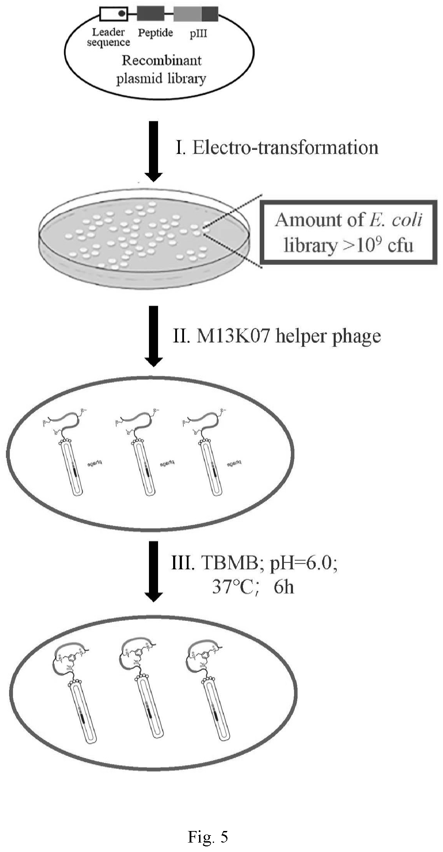

A large number of peptide recombinant phagemids were obtained according to the manner of the step 3. The *E. coli* library was obtained by the electrical conversion method (Fig. 5). The specific steps of electrical conversion were as follows:
(1) The electric shock process of the electric motor was adjusted to Ec2, which represents that *E. coli* was used as the host bacterium. The specification of the electric rotary cup was 0.2cm. The built-in electric shock parameters were as follows: resistance: 200Ω, capacitor: 25µF and voltage: 2.5kV.
(2) 400ng of purified salt-free recombinant phagemid was added to 300µL of the electrocompetent *E. coli* TG1. After mixing homogeneously, the mixture was cooled in ice bath for 1min. The negative control of the enzyme digestion of the Affibody DNA sequence and the enzyme digestion was set.
(3) The mixture of competent *E. coli* and plasmid was transferred to a pre-cooled electric shock cup. The bottom of the electric shock cup was tapped slightly, so that the mixed liquid covered the bottom of the cup without bubbles. Otherwise, an electric arc would be generated at an instantaneous high pressure, which would seriously affect the electric conversion effect.
(4) The electric shock cup was wiped dry to ensure that the outer surface was dried and placed in the electric converter. The pulse was started. When the voice of electric shock completion was heard, the electric shock cup was taken out immediately. 1mL of SOC medium was added into the electric shock cup after the completion of the electric shock. After mixing homogeneously, the mixture was transferred to the centrifuge tube.
(5) The mixture was cultured at 270rpm and 37°C for 1h. The same type of electrotransformation culture solution was combined. 1µL of the culture solution of the experimental group was diluted in series (10⁻³ to 10⁻⁸). 100µL of bacterial solution (three parallel) was inoculated on the 2×YTE/A100/G2 flat plate for calculating the total number of colonies (the number of competent cells was much larger than that of the recombinant plasmid and therefore the number of the recombinant phagemid particles per competent *E. coli* was less than 1).
(6) The remaining culture solution was inoculated on three 150mm 2×YTE/A100/G2 plates and cultured overnight at 37°C.
(7) 5 mL of 2×YT/A100/G2 liquid medium was added to the flat plate. The bacteria on the plate were carefully scraped and collected. 5mL of liquid medium was added again and the collection was repeated. Finally, glycerol with a final concentration of 20% was added. The mixture was stored at -80°C in a refrigerator.
(8) Quality identification of primary *E. coli* library

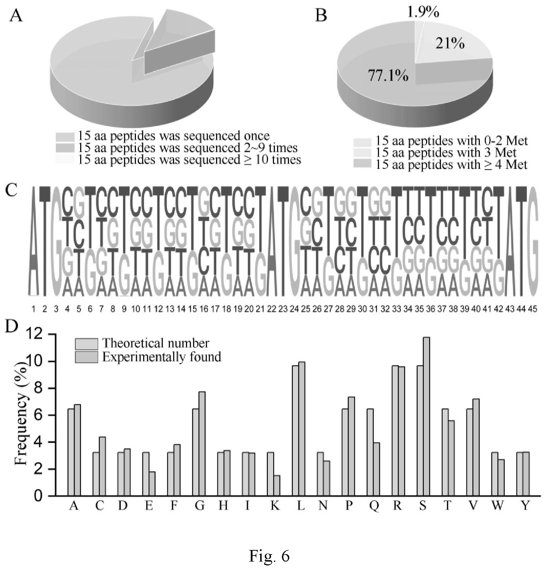

More than 100 monoclonal bacteria were randomly selected and detected by PCR. The *E. coli* library was subsequently subjected to high-throughput sequencing to confirm the diversity of the library (Fig. 6).

### 5. Amplification of helper phage M13K07

(1) The purchased helper phage was subjected to gradient dilution with 2×YT liquid medium and 10 gradient dilutions were obtained. Phage of 10µL of each dilution gradient were taken respectively and added to 200µL of the newly cultured *E. coli* TG1 bacterial solution with OD₆₀₀ of about 0.4. The infection was carried out in a water bath at 37°C for 30min. The water bath was not shaken during the water bath period.
(2) During incubation in the water bath, ten 2×YTE plates were prepared to be used. 100µL of the above infection solution was pipetted. 3 mL of warmed semi-solid 2×YT medium was added. After mixing homogeneously, the mixture was quickly poured into the 2×YTE plate. The plate was cultured overnight in a constant temperature incubator at 37°C.
(3) A small plaque was picked, inoculated into 5mL of newly cultured *E. coli* TG1 bacterial solution with OD₆₀₀ of about 0.4 and incubated at 37°C for 2h. The culture solution was added to 500mL of 2×YT liquid medium, shaken and cultured at 37°C for 1h. Then kanamycin with a final concentration of 50µg/mL was added to the medium. The medium was shaken overnight at 30°C.
(4) The overnight culture solution was centrifuged at 4000rpm for 15min. The supernatant was taken and 100 mL of pre-cooled PEG/NaCl solution was added to 400mL of supernatant. The resultant mixture was allowed to stand on ice for at least 1h and centrifuged at 4°C and 4000rpm for 30min after standing.
(5) The supernatant was removed after centrifugation. The precipitate was suspended with 8mL of PBS. 2mL of PEG/NaCl solution was added. After mixing homogeneously, the mixture was cooled in an ice bath for 20 min and centrifuged at 4°C and 4000rpm for 30min. The PEG/NaCl solution was completely removed by a pipette gun.
(6) The precipitate was suspended with 10mL of PBS. The suspension was sub-packaged in a microcentrifuge tube and centrifuged at 13000rpm for 10min to completely remove the bacterial cells and fragments. The supernatant containing the helper phage was then briefly stored at 4°C or glycerol with a final concentration of 15% was added in the supernatant for a long time storage at 80°C.
(7) Determination of concentration of helper phage M13K07

After the amplification of the helper phage, the concentration needed to be determined.
(1) 1µL of the prepared helper phage was subjected to gradient dilution with 2×YT liquid medium. 10 gradient solutions were obtained. 1µL of each gradient solution of phase was taken, respectively and added to 500µL of newly cultured *E. coli* TG1 bacterial solutions with OD₆₀₀ of about 0.4. The infection was carried out in a water bath at 37°C for 30min. The water bath was not shaken during infection.
(2) During the performance of water bath, 30 2×YTE plates were prepared to be used. 30µL of the above infection solution was pipetted. 3mL of warmed semi-solid 2×YT medium was added to the infection solution, respectively. After mixing homogeneously, the mixture was quickly poured into a 2×YTE flat plate. 3 parallel experiments were carried out in each group. The plate was incubated overnight at 37°C under a constant temperature incubator.
(3) Counting plaque and calculating phage titer

As there were many helper phage that needed to be assisted in the experiment, the amplification method of the helper phage was subsequently used for multiple preparations.

### 6. Preparation of the Phage Library

The primary *E. coli* library must be infected with the helper phage M13K07 to release phages displaying the fusion proteins. The specific procedure for preparing the phage library is as follows (Figure 5):
(1) The E. coli library was added to 200 mL of 2×YT/A100/G2 medium. The medium was incubated with shaking at 37°C for approximately 12 hours until the OD₆₀₀ reached approximately 0.4 to 0.6 (logarithmic phase).
(2) 50 mL of the above culture was taken and 2 × 10¹⁰ pfu of helper phage was added. The resultant medium was incubated in a water bath at 37°C without shaking for 30 minutes.
(3) Following the water bath, the medium was centrifuged at 3600 rpm for 10 minutes and the supernatant was discarded. The precipitate was suspended in 100 mL of 2×YT/A100/K50/G0.1 medium. The resultant medium was incubated with shaking at 30°C overnight.
(4) The remaining culture from Step (2) was incubated with shaking at 37°C for an additional 2 hours. The culture was centrifuged at 4000 rpm for 10 minutes. The precipitate was suspended in 10 mL of 2×YT medium containing 15% glycerol. The resultant mixture was subpackaged into 500 µL EP tubes and stored at -80°C.
(5)The overnight culture from Step 3 was collected and centrifuged at 4000 rpm for 30 minutes at 4°C. 40 mL of the supernatant was transferred into two sterile 50 mL centrifuge tubes. 10 mL of PEG/NaCl solution was added to each tube. The solution was mixed thoroughly and incubated on ice for at least 1 hour.
(6) The resultant mixture was centrifuged at 4000 rpm for 30 minutes at 4°C. The supernatant was discarded and all PEG/NaCl solutions were removed. The phage pellet was suspended in 2 mL of sterile PBS. The sample solution was subpackaged into 1.5 mL EP tubes (1 mL per tube) and centrifuged at 13000 rpm for 10 minutes to remove bacterial cells and cell debris. The supernatant was transferred to a sterile centrifuge tube and stored temporarily at 4°C. For long-term storage, 15% glycerol was added and the resultant was stored at -80°C. This constitutes the primary phage display library.

### 7. Construction of the sulfonium stapled peptide library displayed by phage

The primary phage display library was taken and reacted with 10 µM 1,3,5-tris(bromomethyl)benzene (TBMB) in an acidic buffer (70% PBS, pH 6.0, 30% acetonitrile) at 37°C for 24 hours. The peptides at the N-terminus of the phage coat protein formed sulfonium stapled bicyclic peptides, thereby giving the sulfonium stapled bicyclic peptide library displayed by phage (Fig. 5).

### 8. Counting of the phage library

The prepared phage library must be titrated to determine its potency. Since the genetic material within the phage library was recombinant phagemids, the phages cannot assemble into infectious particles independently. Therefore, infecting *E. coli* TG1 did not yield traditional plaques. Consequently, the counting method for a phage library differed from standard phage counting. It required infecting *E. coli* TG1 followed by plating on ampicillin-containing plate. The phage library count was determined by counting the resulting colonies. The specific procedure was as follows:
(1) A 10-fold serial dilution of the phage display library from 10⁻¹ to 10⁻¹² was carried out. Specifically, 10 µL of the library was added to 90 µL of 2×YT, then 10 µL of that dilution was taken and added to another 90 µL of 2×YT. The same procedure was repeated 12 times. 10 µL of each dilution was added to an EP tube containing 90 µL of *E. coli* TG1 (OD₆₀₀ = 0.4) and the tube was incubated in a water bath at 37°C for 30 minutes. 10 µL of the infected solution was plated onto 2×YTE/A100/G1 solid medium plates (in triplicate) and incubated at 30°C overnight.
(2) The colonies were counted on the plates the following day to calculate the concentration of the phage library.

### Example 4

### Screening of Peptide Inhibitors Targeting SARS CoV-2 PLpro

### 1. Expression, Purification and Biotinylation of Target Protein (PLpro)

The single colonies were picked and transferred to 5mL of the corresponding antibiotic-containing medium. The medium was shaken and incubated overnight at 37°C. The bacterial solution was poured into the corresponding antibiotic-containing medium (1L). The medium was shaken and incubated at 37°C until the OD₆₀₀ value was 0.6 to 0.8. The incubation was stopped. IPTG was added. The culture was induced at 16°C for 16 to 18 hours. The bacteria were collected by centrifugation and re-suspended in PBS. An appropriate amount of protease inhibitor was added for ultrasonic lysis. After lysis, the bacterial solution was centrifuged at 1800rpm for 50 min. The supernatant was filtered with 0.45µM filter membrane. The protein with His tag was first purified by His Trap^{TM} FF Crude column. The column was equilibrated with ultrapure water and protein buffer. The protein solution was subjected to column three times. The impure protein was washed away with a protein buffer with a low concentration of imidazole (less than 50mM). Then the imidazole concentration was gradually increased for collection. The detection was performed with Coomassie Blue G250 during the period. The collected protein solution was subject to ultrafiltration concentration with a corresponding ultrafiltration tube and further to purification with molecular sieves. The purified protein was biotinylated.

### 2. First round screening of phage

The target protein was diluted with PBS to 5µg/mL, formulated for 100µL and coated with streptavidin magnetic beads at 37°C for at least 3h (or overnight at 4°C). The coat buffer was discarded. The streptavidin magnetic beads were washed three times with PBS solution. Then 100 ul of PBS solution containing 2% skim milk powder was added. The mixture was allowed to stand in an incubator at 37°C for 3 h (or closed overnight at 4°C). The streptavidin magnetic beads were then washed 3 times with PBS. A phage library of about 10¹¹ to 10¹²pfu was added to the MPBS buffer. The mixture was allowed to stand in an incubator at 37°C for 2h. The phage library was discarded, washed with PBST for 5 times and washed with PBS for 5 times. 100µL of a strong acid solution (pH=2.2) was added to the streptavidin magnetic bead tube. The tube was flipped repeatedly for 10min to elute the phage that specifically binds to the antigen. Then, 34µL of phage neutralized and eluted by 1M Tris-HCl was added into the streptavidin magnetic bead tube. The tube was flipped and mixed homogeneously. The solution in the streptavidin magnetic bead tube was sucked into a 1.5mL EP tube and stored at 4°C. The specific eluted phage and 130µL of the above solution were pipetted to infect 10mL of *E. coli* TG1 solution with OD₆₀₀=0.4. The mixture was incubated in a water bath at 37°C for 45min. 4 mL of *E. coli* TG1 solution with OD₆₀₀=0.4 was added into the streptavidin magnetic bead tube. The tube was incubated in a water bath at 37°C for 45min. The *E. coli* TG1 solutions in the two tubes were then mixed. 100µL of TG 1 bacteria solution was taken out for 10-fold gradient dilution. 100µL of each was coated to 2×YT/A100/G2 medium. The remaining bacterial solution was centrifuged at 4000rpm at 4°C for 10min. The supernatant was discarded. The bacteria were re-suspended with a small amount of 2×YT (500µL) and coated with 3 to 10 large bacterial culture dishes. The bacteria were scraped and collected from the flat plate. The scraped bacteria were dissolved in 5 mL of 2×YT. 100µL of suspension was inoculated into 50mL of 2×YT/A100/G2 medium. 20% glycerol was added into the remaining bacterial solution. The mixture was sub-packaged and stored at -80°C. The above culture solution was incubated in an incubator at 37°C until OD₆₀₀ reached 0.4 to 0.6. Helper phage M13K07 of 20-fold concentration was added. The mixture was allowed to stand at 37°C and incubated for 30min, and centrifuged at 4000rpm for 10min. The supernatant was discarded. The precipitate was suspended in 50mL of 2×YT/A100/K50/G0.1. The mixture was shaken and incubated overnight at 30°C. The phage was then collected and titrated on the second day. The titrated phage was closed to prepare for the second round screening.

### 3. Second round screening of phage

Steps in the second round were almost identical to those in the first round. In specific operations, the target protein could be moderately reduced by 3 to 5µg, and the magnetic beads could be replaced with neutralizing avidin magnetic beads or neutralizing avidin 96-well plates to reduce the adsorption and aggregation of the same magnetic beads.

### 4. Third round screening of phage

Steps in the second round were almost identical to those in the first round. In specific operations, the target protein could be moderately reduced by 1 to 3µg. The magnetic beads could be replaced with streptavidin magnetic beads.

It was focused whether and to what extent the amount of phage screened in each round of screeningw was enriched during the three rounds of screening. The target protein concentration and the amount of phage in different rounds of screening could be adjusted according to specific situations. The three rounds of screening will generally have several tens of to hundreds of enrichment.

**Table 6. Number and enrichment extent of phage obtained in each round of three rounds of screening**

| | First round screening | | Second round screening | | Third round screening | |
|---|---|---|---|---|---|---|
| | PBS | PLpro (10µg) | PBS | PLpro (5µg) | PBS | PLpro (3µg) |
| Input | 1.2×10¹³ | 1.2×10¹³ | 4.0×10¹² | 4.0×10¹² | 1.2×10¹³ | 1.2×10¹³ |
| Recovery | 4.9×10⁷ | 3.9×10⁷ | 3.43×10⁶ | 4.2×10⁶ | 2.35×10⁶ | 2.4×10⁸ |
| Recovery Rate | 4.08×10⁻⁶ | 3.25×10⁻⁶ | 8.5×10⁻⁷ | 1.05×10⁻⁶ | 1.95×10⁻⁷ | 2×10⁻⁵ |
| Enrichment Extent | - | - | - | 1.23 | - | 102 |

### 5. Determination of screened peptide sequences by high-throughput sequencing

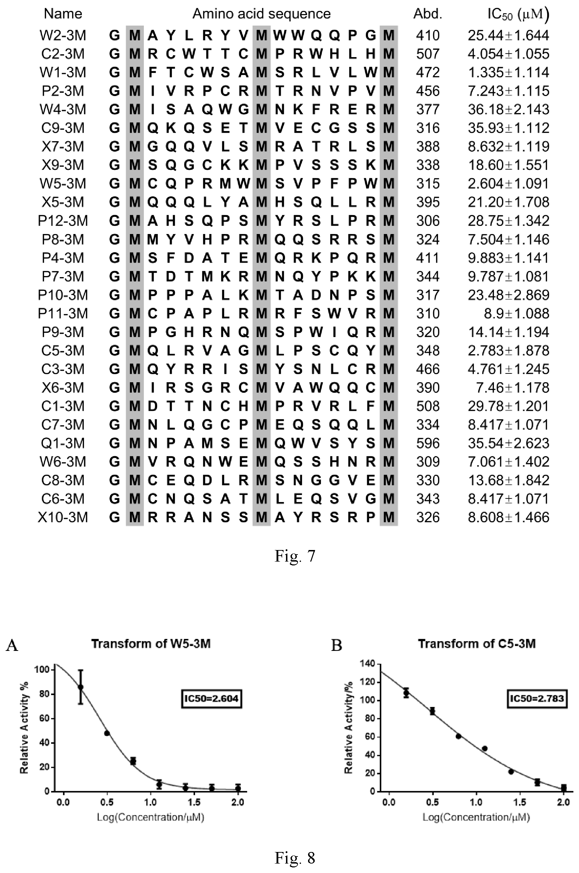

A part of the bacteria were taken out from the enriched phage *E. coli* library in the third round. Plasmid extraction was carried out. PCR was carried out on the random peptide part in the plasmid. The enriched peptide sequence was confirmed by high-throughput sequencing. The peptides obtained by high-throughput sequencing were analyzed. A total of 35 homologous peptide groups were found (Fig. 7).

### Example 5

### Detection of Inhibitory Effect of Sulfonium Salt Stapled Bicyclic Peptide on PLpro Enzyme Activity

In order to determine the inhibition of sulfonium stapled bicyclic peptide inhibitors on protease activity, an LRGG-ACC substrate was used. The inhibitors (0 to 800µM) of different concentrations were mixed with PLpro protein (0.1µM) in experimental buffer (5mM NaCl, 20mM tris, 5mM DTT, pH=8.0). The mixture was reacted at 37°C in a water bath for 1h. Then substrate LGRG-ACC (1µM) was added to the mixture. The fluorescence emission intensity (λEX: 355nm, λEM: 460nm) was measured using the microplate reader in the black-walled 96-well plate. The nonlinear regression analysis was performed using Origin software to obtain a half-inhibition concentration IC₅₀.

**Table 7. Peptide preparation and affinity assay with screened peptides**

| Peptide Names | Peptide Sequence | Sequence Name | IC₅₀ (µM) |
|---|---|---|---|
| P5 | YMVHQSRPMQQLFAQM | SEQ ID NO:9 | N.D. |
| P8 | GMMYVHPRMQQSRRSM | SEQ ID NO:10 | 7.6 |
| P7 | GMTDTMKRMNQYPKKM | SEQ ID NO:11 | 9.787 |
| C1 | YMDTTNCHMPRVRLFM | SEQ ID NO:12 | 29.78 |
| X10 | GMRRANSSMAYRSRPM | SEQ ID NO:13 | 8.608 |
| C5 | GMQLRVAGMLPSCQYM | SEQ ID NO:14 | 2.783 |
| C6 | YMCNQSATMLEQSVGM | SEQ ID NO:15 | 8.417 |
| C7 | YMNLQGCPMEQSQQLM | SEQ ID NO:16 | 4.603 |
| W5 | MCQPRMWMSVPFPWM | SEQ ID NO:17 | 2.604 |
| W6 | GMVRQNWEMQSSHNRM | SEQ ID NO:18 | 7.061 |
| P9 | GMPGHRNQMSPWIQRM | SEQ ID NO:19 | 14.14 |
| P3 | GMPRLLQRMSHMQNCM | SEQ ID NO:20 | 23.58 |
| P4 | GMSFDATEMQRKPQRM | SEQ ID NO:21 | 9.883 |
| X7 | GMGQQVLSMRATRLSM | SEQ ID NO:22 | 8.632 |
| X8 | GMRLRNSPMRRVGILM | SEQ ID NO:23 | N.D. |

Enzyme activity inhibition experiments were carried out with the obtained peptides to detect the activity. The bicyclic peptides W5-3M and C5-3M both showed good enzyme activity inhibition effects, in which the IC₅₀ values were 2.604 and 2.783µM, respectively (Fig. 8).

### Example 6

### Screening of Peptide Inhibitors Targeting Signal Transducer and Activator of Transcription 3 (STAT3)

### 1. Expression, Purification and Biotinylation of Target Protein (STAT3)

The single colonies were picked and transferred to 5mL of the corresponding antibiotic-containing medium. The medium was shaken and incubated overnight at 37°C. The bacterial solution was poured into the corresponding antibiotic-containing medium (1L). The medium was shaken and incubated at 37°C until the OD₆₀₀ value was 0.6 to 0.8. The incubation was stopped. IPTG was added. The culture was induced at 16°C for 16 to 18 hours. The bacteria were collected by centrifugation and re-suspended in PBS. An appropriate amount of protease inhibitor was added for ultrasonic lysis. After lysis, the bacterial solution was centrifuged at 1800rpm for 50 min. The supernatant was filtered with 0.45µM filter membrane. The protein with His tag was first purified by His Trap^{TM} FF Crude column. The column was equilibrated with ultrapure water and protein buffer. The protein solution was subjected to column three times. The impure protein was washed away with a protein buffer with a low concentration of imidazole (less than 50mM). Then the imidazole concentration was gradually increased for collection. The detection was performed with Coomassie Blue G250 during the period. The collected protein solution was subject to ultrafiltration concentration with a corresponding ultrafiltration tube and further to purification with molecular sieves. The purified protein was biotinylated.

### 2. First round screening of phage

The target protein was diluted with PBS to 5µg/mL, formulated for 100µL and coated with streptavidin magnetic beads at 37°C for at least 3h (or overnight at 4°C). The coat buffer was discarded. The streptavidin magnetic beads were washed three times with PBS solution. Then 100 ul of PBS solution containing 2% skim milk powder was added. The mixture was allowed to stand in an incubator at 37°C for 3 h (or closed overnight at 4°C). The streptavidin magnetic beads were then washed 3 times with PBS. A phage library of about 10¹¹ to 10¹²pfu was added to the MPBS buffer. The mixture was allowed to stand in an incubator at 37°C for 2h. The phage library was discarded, washed with PBST for 5 times and washed with PBS for 5 times. 100µL of a strong acid solution (pH=2.2) was added to the streptavidin magnetic bead tube. The tube was flipped repeatedly for 10min to elute the phage that specifically binds to the antigen. Then, 34µL of phage neutralized and eluted by 1M Tris-HCl was added into the streptavidin magnetic bead tube. The tube was flipped and mixed homogeneously. The solution in the streptavidin magnetic bead tube was sucked into a 1.5mL EP tube and stored at 4°C. The specific eluted phage and 130µL of the above solution were pipetted to infect 10mL of *E. coli* TG1 solution with OD₆₀₀=0.4. The mixture was incubated in a water bath at 37°C for 45min. 4 mL of *E. coli* TG1 solution with OD₆₀₀=0.4 was added into the streptavidin magnetic bead tube. The tube was incubated in a water bath at 37°C for 45min. The *E. coli* TG1 solutions in the two tubes were then mixed. 100µL of TG 1 bacteria solution was taken out for 10-fold gradient dilution. 100µL of each was coated to 2×YT/A100/G2 medium. The remaining bacterial solution was centrifuged at 4000rpm at 4°C for 10min. The supernatant was discarded. The bacteria were re-suspended with a small amount of 2×YT (500µL) and coated with 3 to 10 large bacterial culture dishes. The bacteria were scraped and collected from the flat plate. The scraped bacteria were dissolved in 5 mL of 2×YT. 100µL of suspension was inoculated into 50mL of 2×YT/A100/G2 medium. 20% glycerol was added into the remaining bacterial solution. The mixture was sub-packaged and stored at -80°C. The above culture solution was incubated in an incubator at 37°C until OD₆₀₀ reached 0.4 to 0.6. Helper phage M13K07 of 20-fold concentration was added. The mixture was allowed to stand at 37°C and incubated for 30min, and centrifuged at 4000rpm for 10min. The supernatant was discarded. The precipitate was suspended in 50mL of 2×YT/A100/K50/G0.1. The mixture was shaken and incubated overnight at 30°C. The phage was then collected and titrated on the second day. The titrated phage was closed to prepare for the second round screening.

### 3. Second round screening of phage

Steps in the second round were almost identical to those in the first round. In specific operations, the target protein could be moderately reduced by 3 to 5µg, and the magnetic beads could be replaced with neutralizing avidin magnetic beads or neutralizing avidin 96-well plates to reduce the adsorption and aggregation of the same magnetic beads.

### 4. Third round screening of phage

Steps in the second round were almost identical to those in the first round. In specific operations, the target protein could be moderately reduced by 1 to 3µg. The magnetic beads could be replaced with streptavidin magnetic beads.

It was focused whether and to what extent the amount of phage screened in each round of screeningw was enriched during the three rounds of screening. The target protein concentration and the amount of phage in different rounds of screening could be adjusted according to specific situations. The three rounds of screening will generally have several tens of to hundreds of enrichment.

**Table 8. Number and the extent of enrichment of phage obtained in each round of the three rounds of screening**

| | First round screening | | Second round screening | | Third round screening | |
|---|---|---|---|---|---|---|
| | PBS | PLpro (10µg) | PBS | PLpro (5µg) | PBS | PLpro (3µg) |
| Input | 6.6×10¹¹ | 6.6×10¹¹ | 7.2×10¹³ | 7.2×10¹³ | 1.0×10¹⁴ | 1.0×10¹⁴ |
| Recovery | 7.4×10⁵ | 3.04×10⁶ | 1.06×10⁶ | 1.47×10⁷ | 4.88×10⁶ | 4.32×10⁸ |
| Recovery Rate | 1.12×10⁻⁶ | 4.61×10⁻⁶ | 1.47×10⁻⁸ | 2.04×10⁻⁷ | 4.88×10⁻⁸ | 4.32×10⁻⁶ |
| Enrichment Extent | - | 4.12 | - | 13.88 | - | 88.52 |

### 5. Determination of screened peptide sequences by high-throughput sequencing

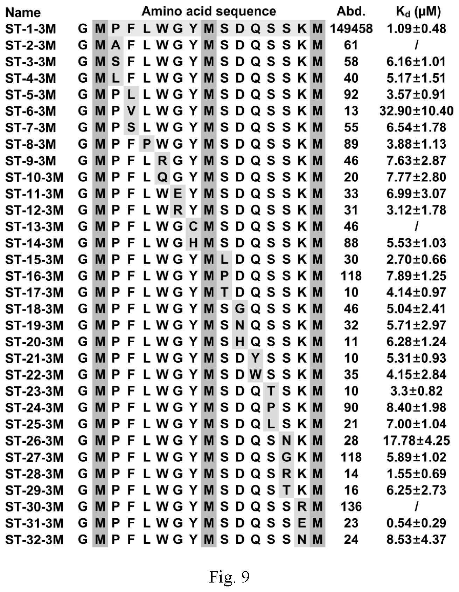

A part of the bacteria were taken out from the enriched phage *E. coli* library in the third round. Plasmid extraction was carried out. PCR was carried out on the random peptide part in the plasmid. The enriched peptide sequence was confirmed by high-throughput sequencing. The peptides obtained by high-throughput sequencing were analyzed. Finally, one homologous peptide group were found (Fig. 9).

### Example 7

### Detection of Affinity of Sulfonium Stapled Bicyclic Peptide and Protein STAT3

The affinity of the sulfonium stapled bicyclic peptide inhibitor and STAT3 protein was determined by the MST method. The purified 10µL STAT3 protein solution (about 100µM) was taken and subjected to a 2-fold gradient dilution to prepare 16 concentration gradient dilutions. The peptides with the FAM tags were diluted to a final concentration of 40nM. 10µL of the protein gradient dilutions was taken for mixing. A mixed sample was sucked with a capillary to perform the MST experiment.

**Table 9. Peptide preparation and affinity assay with screened peptides**

| Peptide Names | Peptide Sequence | Sequence Name | Kd (µM) |
|---|---|---|---|
| ST1 | MPFLWGYMSDQSSKM | SEQ ID NO:24 | 1.09 |
| ST2 | MAFLWGYMSDQSSKM | SEQ ID NO:25 | / |
| ST3 | MSFLWGYMSDQSSKM | SEQ ID NO:26 | 6.16 |
| ST4 | MLFLWGYMSDQSSKM | SEQ ID NO:27 | 5.17 |
| ST5 | MPLLWGYMSDQSSKM | SEQ ID NO:28 | 3.57 |
| ST6 | MPVLWGYMSDQSSKM | SEQ ID NO:29 | 32.90 |
| ST7 | MPSLWGYMSDQSSKM | SEQ ID NO:30 | 6.54 |
| ST8 | MPFPWGYMSDQSSKM | SEQ ID NO:31 | 3.88 |
| ST9 | MPFLRGYMSDQSSKM | SEQ ID NO:32 | 7.63 |
| ST10 | MPFLQGYMSDQSSKM | SEQ ID NO:33 | 7.77 |
| ST11 | MPFLWEYMSDQSSKM | SEQ ID NO:34 | 6.99 |
| ST12 | MPFLWRYMSDQSSKM | SEQ ID NO:35 | 3.12 |
| ST13 | MPFLWGCMSDQSSKM | SEQ ID NO:36 | / |
| ST14 | MPFLWGHMSDQSSKM | SEQ ID NO:37 | 5.53 |
| ST15 | MPFLWGYMLDQSSKM | SEQ ID NO:38 | 2.70 |
| ST16 | MPFLWGYMPDQSSKM | SEQ ID NO:39 | 7.89 |
| ST17 | MPFLWGYMTDQSSKM | SEQ ID NO:40 | 4.14 |
| ST18 | MPFLWGYMSGQSSKM | SEQ ID NO:41 | 5.04 |
| ST19 | MPFLWGYMSNQSSKM | SEQ ID NO:42 | 5.71 |
| ST20 | MPFLWGYMSHQSSKM | SEQ ID NO:43 | 6.28 |
| ST21 | MPFLWGYMSDYSSKM | SEQ ID NO:44 | 5.31 |
| ST22 | MPFLWGYMSDWSSKM | SEQ ID NO:45 | 4.15 |
| ST23 | MPFLWGYMSDQTSKM | SEQ ID NO:46 | 3.3 |
| ST24 | MPFLWGYMSDQPSKM | SEQ ID NO:47 | 8.40 |
| ST25 | MPFLWGYMSDQLSKM | SEQ ID NO:48 | 7.00 |
| ST26 | MPFLWGYMSDQSNKM | SEQ ID NO:49 | 17.78 |
| ST27 | MPFLWGYMSDQSGKM | SEQ ID NO:50 | 5.89 |
| ST28 | MPFLWGYMSDQSRKM | SEQ ID NO:51 | 1.55 |
| ST29 | MPFLWGYMSDQSTKM | SEQ ID NO:52 | 6.25 |
| ST30 | MPFLWGYMSDQSSRM | SEQ ID NO:53 | / |
| ST31 | MPFLWGYMSDQSSEM | SEQ ID NO:54 | 0.54 |
| ST32 | MPFLWGYMSDQSSNM | SEQ ID NO:55 | 8.53 |

The affinity assay was performed on the obtained peptide (Table 9 and Fig. 9). The bicyclic peptides ST1-3M and ST31-3M both showed good affinity effects, in which their Kd values were 1.09 and 0.54µM, respectively (Fig. 9).

### Example 8

### Screening of Peptide Inhibitors Targeting estrogen receptor α (ERα)

### 1. Expression, Purification and Biotinylation of Target Protein (ERα)

The single colonies were picked and transferred to 5mL of the corresponding antibiotic-containing medium. The medium was shaken and incubated overnight at 37°C. The bacterial solution was poured into the corresponding antibiotic-containing medium (1L). The medium was shaken and incubated at 37°C until the OD₆₀₀ value was 0.6 to 0.8. The incubation was stopped. IPTG was added. The culture was induced at 16°C for 16 to 18 hours. The bacteria were collected by centrifugation and re-suspended in PBS. An appropriate amount of protease inhibitor was added for ultrasonic lysis. After lysis, the bacterial solution was centrifuged at 1800rpm for 50 min. The supernatant was filtered with 0.45µM filter membrane. The protein with His tag was first purified by His Trap^{TM} FF Crude column. The column was equilibrated with ultrapure water and protein buffer. The protein solution was subjected to column three times. The impure protein was washed away with a protein buffer with a low concentration of imidazole (less than 50mM). Then the imidazole concentration was gradually increased for collection. The detection was performed with Coomassie Blue G250 during the period. The collected protein solution was subject to ultrafiltration concentration with a corresponding ultrafiltration tube and further to purification with molecular sieves. The purified protein was biotinylated.

### 2. First round screening of phage

The target protein was diluted with PBS to 5µg/mL, formulated for 100µL and coated with streptavidin magnetic beads at 37°C for at least 3h (or overnight at 4°C). The coat buffer was discarded. The streptavidin magnetic beads were washed three times with PBS solution. Then 100 ul of PBS solution containing 2% skim milk powder was added. The mixture was allowed to stand in an incubator at 37°C for 3 h (or closed overnight at 4°C). The streptavidin magnetic beads were then washed 3 times with PBS. A phage library of about 10¹¹ to 10¹²pfu was added to the MPBS buffer. The mixture was allowed to stand in an incubator at 37°C for 2h. The phage library was discarded, washed with PBST for 5 times and washed with PBS for 5 times. 100µL of a strong acid solution (pH=2.2) was added to the streptavidin magnetic bead tube. The tube was flipped repeatedly for 10min to elute the phage that specifically binds to the antigen. Then, 34µL of phage neutralized and eluted by 1M Tris-HCl was added into the streptavidin magnetic bead tube. The tube was flipped and mixed homogeneously. The solution in the streptavidin magnetic bead tube was sucked into a 1.5mL EP tube and stored at 4°C. The specific eluted phage and 130µL of the above solution were pipetted to infect 10mL of *E. coli* TG1 solution with OD₆₀₀=0.4. The mixture was incubated in a water bath at 37°C for 45min. 4 mL of *E. coli* TG1 solution with OD₆₀₀=0.4 was added into the streptavidin magnetic bead tube. The tube was incubated in a water bath at 37°C for 45min. The *E. coli* TG1 solutions in the two tubes were then mixed. 100µL of TG 1 bacteria solution was taken out for 10-fold gradient dilution. 100µL of each was coated to 2×YT/A100/G2 medium. The remaining bacterial solution was centrifuged at 4000rpm at 4°C for 10min. The supernatant was discarded. The bacteria were re-suspended with a small amount of 2×YT (500µL) and coated with 3 to 10 large bacterial culture dishes. The bacteria were scraped and collected from the flat plate. The scraped bacteria were dissolved in 5 mL of 2×YT. 100µL of suspension was inoculated into 50mL of 2×YT/A100/G2 medium. 20% glycerol was added into the remaining bacterial solution. The mixture was sub-packaged and stored at -80°C. The above culture solution was incubated in an incubator at 37°C until OD₆₀₀ reached 0.4 to 0.6. Helper phage M13K07 of 20-fold concentration was added. The mixture was allowed to stand at 37°C and incubated for 30min, and centrifuged at 4000rpm for 10min. The supernatant was discarded. The precipitate was suspended in 50mL of 2×YT/A100/K50/G0.1. The mixture was shaken and incubated overnight at 30°C. The phage was then collected and titrated on the second day. The titrated phage was closed to prepare for the second round screening.

### 3. Second round screening of phage

Steps in the second round were almost identical to those in the first round. In specific operations, the target protein could be moderately reduced by 3 to 5µg, and the magnetic beads could be replaced with neutralizing avidin magnetic beads or neutralizing avidin 96-well plates to reduce the adsorption and aggregation of the same magnetic beads.

### 4. Third round screening of phage

Steps in the second round were almost identical to those in the first round. In specific operations, the target protein could be moderately reduced by 1 to 3µg. The magnetic beads could be replaced with streptavidin magnetic beads.

It was focused whether and to what extent the amount of phage screened in each round of screeningw was enriched during the three rounds of screening. The target protein concentration and the amount of phage in different rounds of screening could be adjusted according to specific situations. The three rounds of screening will generally have several tens of to hundreds of enrichment.

**Table 10. Number and enrichment extent of phage obtained in each round of the three rounds of screening**

| | First round screening | | Second round screening | | Third round screening | |
|---|---|---|---|---|---|---|
| | PBS | PLpro (10µg) | PBS | PLpro (5µg) | PBS | PLpro (3µg) |
| Input | 5.6×10¹² | 5.6×10¹² | 1×10¹³ | 1×10¹³ | 1.0×10¹³ | 1.0×10¹³ |
| Recovery | 1.08×10⁷ | 8.4×10⁶ | 1.896×10⁶ | 3.492×10⁷ | 3.5×10⁶ | 6.24×10⁷ |
| Recovery Rate | 1.9×10⁻⁶ | 1.5×10⁻⁶ | 1.896×10⁻⁸ | 3.492×10⁻⁶ | 3.5×10⁻⁷ | 6.24×10⁻⁶ |
| Enrichment Extent | - | - | - | 18.41 | - | 17.82 |

### 5. Determination of screened peptide sequences by high-throughput sequencing

A part of the bacteria were taken out from the enriched phage *E. coli* library in the third round. Plasmid extraction was carried out. PCR was carried out on the random peptide part in the plasmid. The enriched peptide sequence was confirmed by high-throughput sequencing. The peptides obtained by high-throughput sequencing were analyzed. Finally, 20 homologous peptide groups were found.

### Example 9

### Detection of Affinity of Sulfonium Stapled Bicyclic Peptide and Protein ERα

The affinity of the sulfonium stapled bicyclic peptide inhibitor and ERα protein was determined by the MST method. The purified 10µL STAT3 protein solution (about 100µM) was taken and subjected to a 2-fold gradient dilution to prepare 16 concentration gradient dilutions. The peptides with the FAM tags were diluted to a final concentration of 40nM. 10µL of the protein gradient dilutions was taken for mixing. A mixed sample was sucked with a capillary to perform the MST experiment.

**Table 11. Peptide preparation and affinity assay with screened peptides**

| Peptide Names | Peptide Sequence | Sequence Name | Kd (µM) |
|---|---|---|---|
| ER-C1-3M | GMCPWYKGMVCSRFVM | SEQ ID NO:56 | 1.11 |
| ER-P1-3M | YMPTSLQFMAGQRHPM | SEQ ID NO:57 | 8.44 |
| ER-P2-3M | GMYVVLQRMYRPRNVM | SEQ ID NO:58 | 0.93 |
| ER-R1-3M | GMRISQTQMQDSWDWM | SEQ ID NO:59 | 3.05 |
| ER-R2-3M | GMRLAVTTMGYGFLQM | SEQ ID NO:60 | 0.26 |
| ER-P3-3M | GMIRYPSTMLDRPRQM | SEQ ID NO:61 | 7.53 |
| ER-P4-3M | YMLRPQQVMRLFATSM | SEQ ID NO:62 | 2.21 |
| ER-S1-3M | GMSLCAHPMAGVPSYM | SEQ ID NO:63 | 0.64 |
| ER-R4-3M | YMDCLCPRMRSFAGAM | SEQ ID NO:64 | 0.38 |
| ER-X1-3M | YMPIARKSMRHGRSSM | SEQ ID NO:65 | 0.53 |
| ER-R5-3M | YMSVRDADMRQRGASM | SEQ ID NO:66 | 5.28 |
| ER-X2-3M | YMLAKQYPMRQASCSM | SEQ ID NO:67 | 0.36 |
| ER-P5-3M | YMQATFPSMRGLQCDM | SEQ ID NO:68 | 0.55 |
| ER-R7-3M | GMARGRGQMQFYTSSM | SEQ ID NO:69 | 0.91 |
| ER-S2-3M | YMSPKLIAMGLQTPIM | SEQ ID NO:56 | 0.42 |
| ER-S3-3M | YMSPRNGHMLRNSSDM | SEQ ID NO:70 | 0.37 |
| ER-V2-3M | YMVRGRAQMSPALVQM | SEQ ID NO:71 | 10.37 |
| ER-W1-3M | GMAQQAKWMIRWLLGM | SEQ ID NO:72 | 5.11 |
| ER-R9-3M | GMRRTYYRMRRAPQLM | SEQ ID NO:73 | 1.09 |
| ER-W3-3M | GMCGSLQPMPWLARYM | SEQ ID NO:74 | 1.03 |

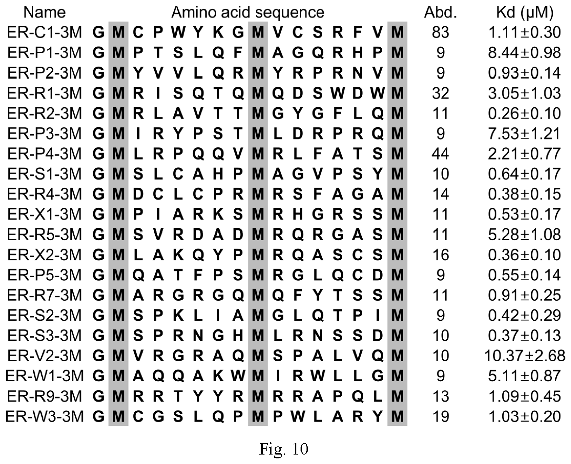

The affinity assay was performed on the obtained peptide (Table 11 and Fig. 10). The bicyclic peptides ER-R2-3M and ER-X2-3M both showed good affinity effects, in which their Kd values were 0.26 and 0.36µM, respectively (Fig. 10).

In the present disclosure, relational terms, such as first and second and the like, are used solely to distinguish one entity or operation from another entity or operation without necessarily requiring or implying any such actual relationship or order between such entities or operations.

From the foregoing it will be appreciated that, although specific embodiments of the present disclosure have been described herein for illustrative purposes, various modifications or improvements may be made by those skilled in the art without departing from the spirit and scope of the present disclosure. Such variations or modifications are intended to fall within the scope of the claims appended hereto.

## Claims

1. A phage particle complex, comprising: a peptide displayed on a surface of the phage, a nucleic acid sequence encoding the displayed peptide inside the phage, and a linking compound, wherein the linking compound forms a bicyclic peptide comprising at least three discrete ionic bonds with the peptide.

2. The phage particle complex of claim 1, wherein the linking compound has molecular symmetry corresponding to the number of the ionic bonds.

3. The phage particle complex of claim 1 or 2, wherein the linking compound has triple molecular symmetry and the linking compound is linked to the peptide by three discrete ionic bonds.

4. The phage particle complex of any one of claims 1 to 3, wherein the linking compound comprises a chemical group of a rigid structure, preferably comprising a benzene ring structure or a cycloalkene structure, more preferably 1,3,5-tribromobenzene, 1,3,5-tris(bromomethyl)-2,4,6-triethylbenzene or 1,3,5-tris(bromomethyl)cyclohexene.

5. The phage particle complex of any one of claims 1 to 4, wherein the peptide comprises at least three methionine residues, preferably a discrete ionic bond is formed by linking the compound with methionine in the peptide.

6. The phage particle complex of any one of claims 1 to 5, wherein a peptide sequence comprises MXmMXnM, wherein X represents a natural amino acid residue, M represents methionine, m is an integer selected from 2 to 20, and n is an integer selected from 2 to 20.

7. The phage particle complex of any one of claims 1 to 6, wherein the bicyclic peptide has a structure of: 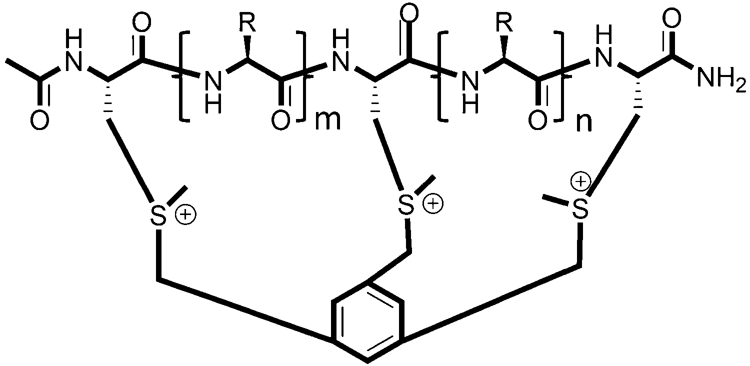 wherein R is selected from a side chain of 20 natural amino acids, m is an integer selected from 2 to 20, and n is an integer selected from 2 to 20.

8. The phage particle complex of any one of claims 1 to 6, wherein the bicyclic peptide is selected from the group consisting of SEQ ID NO:9 to SEQ ID NO:23.

9. A peptide library, comprising the phage particle complex of any one of claims 1 to 8.

10. A process for preparing a phage particle complex, comprising forming a bicyclic peptide containing at least three discrete ionic bonds by a peptide displayed on a surface of the phage with a linking compound.

11. The process of claim 10, wherein the bicyclic peptide is formed in a polar solvent, preferably the polar solvent is selected from the group consisting of an aqueous solution of acetonitrile, an aqueous solution of methanol and any mixture thereof.

12. The process of claim 10 or 11, wherein the bicyclic peptide is formed at 4 to 40°C.

13. The process of any one of claims 10 to 12, wherein the bicyclic peptide is formed at a pH of 3 to 10.

14. The process of any one of claims 10 to 13, wherein reaction time of the peptide and the linking compound to form the bicyclic peptide is from 1 to 36 hours.

15. The process of any one of claims 10 to 14, wherein the linking compound comprises a chemical group of a rigid structure, preferably comprising a benzene ring structure or a cycloalkene structure, more preferably 1,3,5-tribromobenzene, 1,3,5-tris(bromomethyl)-2,4,6-triethylbenzene, or 1,3,5-tris(bromomethyl)cyclohexene.

16. The process of any one of claims 10 to 15, wherein the bicyclic peptide has a structure of: 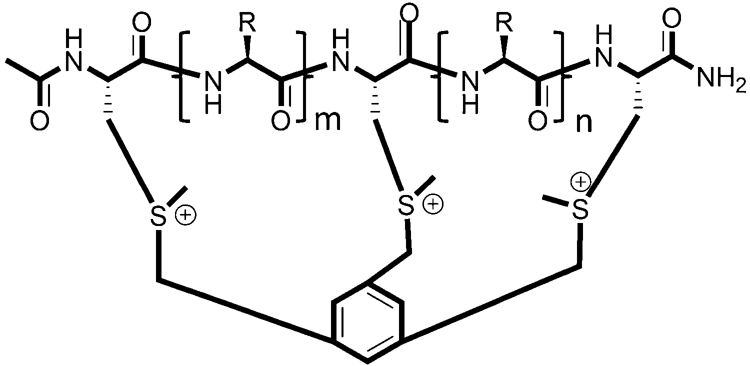 wherein R is selected from a side chain of 20 natural amino acids, m is an integer selected from 2 to 20, and n is an integer selected from 2 to 20.

17. The process of any one of claims 10 to 16, wherein the bicyclic peptide is selected from the group consisting of SEQ ID NO:9 to SEQ ID NO:23.

18. A phage particle complex prepared by the process of any one of claims 10 to 17.

19. The phage particle complex of claim 18, further comprising a nucleic acid sequence encoding the peptide.

20. The phage particle complex of claim 18 or 19, wherein the peptide comprises at least three methionine residues, preferably a discrete ionic bond is formed by linking the compound with methionine in the peptide.

21. Use of the peptide library of claim 9 in manufacture of a peptide inhibitor.

22. The use of claim 21, wherein the peptide inhibitor is selected from the group consisting of an infectious disease peptide inhibitor, a tumor peptide inhibitor, an endocrine disease peptide inhibitor and a digestive system disease peptide inhibitor.

23. The use of claim 21, wherein the peptide inhibitor is selected from the group consisting of a SARS-CoV-2 PLpro peptide inhibitor, a breast cancer peptide inhibitor, a leukemia peptide inhibitor, a nutritional and metabolic disease peptide inhibitor and an enteritis peptide inhibitor.

24. The use of any one of claims 21 to 23, wherein the peptide inhibitor is selected from the group consisting of SEQ ID NO:9 to SEQ ID NO:76.

25. A nucleotide sequence selected from the group consisting of SEQ ID NO:1 to SEQ ID NO:8.
